(19) **Europäisches Patentamt / European Patent Office / Office européen des brevets**

(11) **EP 1 552 793 B1**

(12) **FASCICULE DE BREVET EUROPEEN**

(45) Date de publication et mention
de la délivrance du brevet:
**28.02.2007 Bulletin 2007/09**

(51) Int Cl.:
*A61B 17/34* *(2006.01)*    *A61B 19/00* *(2006.01)*

(21) Numéro de dépôt: **04290028.2**

(22) Date de dépôt: **07.01.2004**

(54) **Dispositif de trocart pour le passage d'un instrument chirurgical**

Trocar device for the passage of a surgical instrument

Trokar für die Durchführung eines chirurgischen Instrumentes

(84) Etats contractants désignés:
**AT BE BG CH CY CZ DE DK EE ES FI FR GB GR HU IE IT LI LU MC NL PT RO SE SI SK TR**

(43) Date de publication de la demande:
**13.07.2005 Bulletin 2005/28**

(73) Titulaire: **UNIVERSITE PIERRE ET MARIE CURIE 75252 Paris Cédex 05 (FR)**

(72) Inventeurs:
• **Morel, Guillaume**
  **77580 Maisoncelles en Brie (FR)**
• **Zemiti, Nabil**
  **92260 Fontenay aux Roses (FR)**

(74) Mandataire: **Wagret, Frédéric**
  **Cabinet Wagret,**
  **19, rue de Milan**
  **75009 Paris (FR)**

(56) Documents cités:
**EP-A- 0 548 872**    **EP-A- 0 624 346**
**EP-A- 1 285 634**    **WO-A-01/70117**

• **WAGNER, C.R., STYLOPOULOS, N., JACKSON, P.G, HOWE, R.D.: "The Benefit of Force Feedback in Surgery: Examination of Blunt Dissection" 13 juin 2003 (2003-06-13) , IEEE TRANSACTIONS ON ROBOTICS, 2003 , HARVARD UNIVERSITY, USA XP002280912 ISBN: 0-7695-1489-8 * le document en entier ***
• **ATI INDUSTRIAL AUTOMATION: "Multi-Axis Force/Torque Sensor F/T" 11 avril 2003 (2003-04-11) , ATI INDUSTRIAL AUTOMATION 2003 CATALOG , APEX, NC 27539 USA XP002280639 * page 12 - page 13 ***
• **WAGNER C R ET AL: "The role of force feedback in surgery: analysis of blunt dissection" , HAPTIC INTERFACES FOR VIRTUAL ENVIRONMENT AND TELEOPERATOR SYSTEMS, 2002. HAPTICS 2002. PROCEEDINGS. 10TH SYMPOSIUM ON ORLANDO, FL, USA 24-25 MARCH 2002, LOS ALAMITOS, CA, USA,IEEE COMPUT. SOC, US, PAGE(S) 68-74 XP010590539 ISBN: 0-7695-1489-8 * le document en entier ***

EP 1 552 793 B1

**Description**

La présente invention concerne un dispositif de trocart pour le passage d'un instrument chirurgical.

La laparoscopie opératoire consiste à pratiquer des interventions chirurgicales à l'aide d'instruments de chirurgie miniaturisés dont le faible diamètre permet leur passage à travers des trocarts, qui sont des tubes creux, insérés à travers la paroi abdominale ou thoracique d'un patient.

Plus précisément, la laparoscopie consiste à introduire, d'une part, un laparoscope dans la paroi abdominale ou thoracique d'un patient permettant ainsi au chirurgien de regarder et examiner, et d'autre part, des instruments permettant d'effectuer une intervention sous contrôle visuel via le laparoscope sans pour autant avoir à ouvrir tout l'abdomen.

Bien que pouvant être entièrement réalisée à la main, une opération laparoscopique est parfois réalisée par un système robotique.

Dans ce cas, afin d'améliorer la précision de la laparoscopie, le chirurgien ne manipule pas directement les outils chirurgicaux mais le fait à partir d'une interface électro-mécanique.

Ainsi, le chirurgien manoeuvre, à partir d'une interface, des bras de commande qui commandent des bras robotisés agissant directement sur le patient, ces bras robotisés étant reliés à des outils chirurgicaux ou un laparoscope par exemple.

Toutefois, un problème rencontré lors de l'utilisation de ces systèmes robotisés est que le chirurgien ne peut pas estimer de manière directe les efforts appliqués par le laparoscope ou les instruments sur les organes internes du patient.

De ce fait, il doit compenser le manque de sensation tactile par une estimation visuelle des déformations des organes, observées sur l'écran de visualisation de l'image laparoscopique.

Cela est particulièrement gênant dans le cas d'opérations endochirurgicales qui nécessitent des gestes microchirurgicaux très précis et où tous les paramètres de mesure doivent être connus.

A l'heure actuelle, pour des applications classiques (non endoscopiques), il existe des systèmes de commande téléopérés permettant un contrôle des efforts appliqués par l'opérateur sur le patient.

Toutefois, ces méthodes sont basées sur l'hypothèse que l'on peut mesurer ou estimer l'interaction que l'on souhaite ressentir.

Cela est difficilement envisageable en chirurgie endoscopique puisqu'il faudrait, dans ce cas, intégrer à l'intérieur du patient un capteur d'efforts satisfaisant les contraintes de stérilisation, d'encombrement, de précision et de coût.

Il serait ainsi particulièrement avantageux de pouvoir estimer précisément la force d'interaction instrument/organe interne, tout en n'utilisant pas de capteur interne.

Il est connu selon la demande de brevet EP 0 624 346 de réaliser un dispositif de trocart à ultrason avec une pointe biseautée, ce dispositif étant utilisé pour apprécier, à l'aide d'un capteur d'effort, les forces de pénétration dans le tissu lors de l'insertion du trocart dans un patient. Il n'est donc pas utilisé pour mesurer l'effort exercé sur les organes internes d'un patient, tout en satisfaisant les conditions de stérilisations nécessaires, lors d'une opération chirurgicale.

La présente invente se propose de résoudre ce problème à l'aide d'un dispositif instrumentalisé simple, peu onéreux, fiable et pouvant être installé sur des systèmes téléopérés robotisés déjà existants.

La présente invention concerne un dispositif de trocart pour le passage d'un instrument chirurgical, caractérisé en ce qu'il comporte des moyens de mesure de l'effort exercé par ledit instrument sur les organes internes d'un patient, lesdits moyens de mesure se présentant sous la forme d'au moins un capteur d'efforts disposé entre le trocart et un guide.

De manière plus précise, le capteur d'effort est monté sur le trocart et est avantageusement conformé en galet avec un orifice central.

Avantageusement, le guide se présente sous la forme d'un élément tubulaire d'axe longitudinal (X-X) comportant une plaque circulaire, perpendiculaire à (X-X), à une de ses extrémités et est inséré dans ledit orifice central dudit capteur d'efforts et ledit dispositif de trocart.

Selon une première forme de réalisation du dispositif de trocart selon l'invention, l'instrument est mis en mouvement par un bras robotisé et un second capteur d'efforts est disposé entre l'extrémité du bras robotisé et l'instrument chirurgical.

Selon une seconde forme de réalisation, l'instrument est mis en mouvement par un translateur disposé sur le guide, préférentiellement par un translateur à galet et le dispositif de trocart est mis en mouvement par l'extrémité d'un bras robotisé.

Avantageusement, de manière générale, le déplacement du bras robotisé est commandé à partir d'une interface.

La présente invention est maintenant décrite à partir d'exemples uniquement illustratifs et nullement limitatifs de la portée de l'invention et à l'aide des illustrations ci-jointes dans lesquelles :

- La figure 1 représente une vue schématique d'un ensemble de manipulation endochirurgicale téléopérée ;
- La figure 2 représente une vue éclatée en perspective d'un dispositif de trocart selon l'invention où l'instrument chirurgical est déplacé par un bras robotisé, et

- La figure 3 représente une vue éclatée en perspective d'un dispositif de trocart selon l'invention, où l'instrument chirurgical est déplacé par un translateur.

La présente invention est décrite pour une utilisation lors d'une opération chirurgicale du type laparoscopie, étant entendu que le principe général de l'invention peut être appliqué notamment à tout type d'opération chirurgicale téléopérée où un trocart est utilisé, ou encore à tout système d'entraînement et d'initiation au geste chirurgical destiné à la formation des chirurgiens.

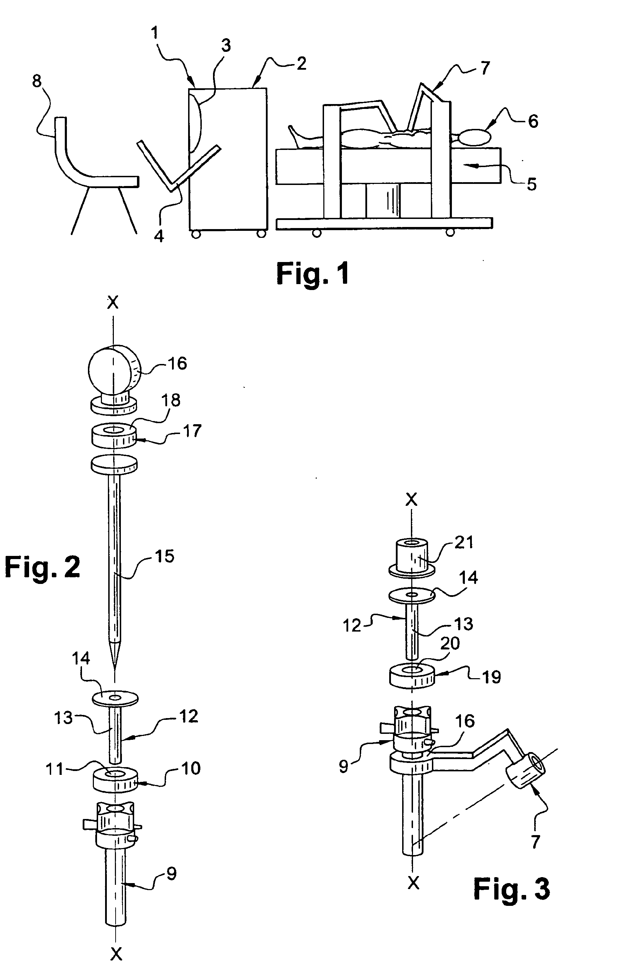

La figure 1 représente un système robotisé 1 permettant la réalisation d'une opération chirurgicale téléopérée à partir d'une interface 2, et plus précisément pour la réalisation d'opérations endochirurgicales.

L'interface 2 se présente sous la forme d'un écran de visualisation 3 et d'une paire de bras de commande 4 apte à être manipulée par un chirurgien.

A l'interface 2 est associée une table opératoire 5 sur laquelle est disposé le patient 6 devant être opéré.

A la table opératoire 5 est associé un ensemble de bras robotisés 7, étant entendu qu'à un bras robotisé peut être associé un laparoscope, une caméra, un jeu de pinces, un scalpel, etc...

De manière avantageuse, le déplacement de la paire de bras de commande 4 par le chirurgien entraîne le déplacement des bras robotisés 7, étant entendu que plusieurs bras robotisés 7 peuvent être commandés par la paire de bras de commande 4, l'interface 2 permettant la sélection des bras robotisés 7 que le chirurgien souhaite téléguider.

De manière avantageuse, l'interface 2 comporte un siège 8 permettant d'améliorer le confort du chirurgien lors de l'opération et de diminuer la fatigue occasionnée par une position debout prolongée durant l'opération.

La figure 2 représente une vue éclatée en perspective d'un dispositif de trocart associé à un instrument mis en mouvement par un bras robotisé.

Avantageusement, un trocart 9 de type connu en soi est utilisé, c'est-à-dire qu'il se présente sous la forme d'un élément tubulaire creux et est inséré dans la paroi abdominale d'un patient 6 lors de l'opération chirurgicale.

Sur le trocart 9 est monté un premier capteur d'effort 10, de type connu en soi et commercialement disponible, par exemple un capteur connu sous le nom ATI Nano43 (marque déposée).

Le premier capteur d'effort 10 est de forme cylindrique, de préférence sous la forme d'un galet et présente un orifice central 11 dans lequel est apte à s'insérer un guide 12 passif et étanche en translation.

Le guide 12 se présente sous la forme d'un élément tubulaire creux 13 présentant à une de ses extrémités une plaque circulaire 14 disposée transversalement à l'axe longitudinal (X-X) de l'élément tubulaire 13.

De manière avantageuse, l'élément tubulaire 13 s'insère dans l'orifice central 11 du premier capteur d'effort et dans le trocart 9.

Le guide 12 est avantageusement réalisé en un matériau stérilisable, par exemple en acier inoxydable.

Afin de rendre l'ensemble guide 12 et premier capteur d'effort 10 étanche, un joint en caoutchouc de type connu en soi est ajouté entre ces deux éléments (non représenté sur la figure mais de type connu en soi).

Un instrument 15, par exemple un laparoscope, relié à l'extrémité 16 d'un bras robotisé 7 est apte à coulisser dans le guide 12 selon un ou deux degrés de liberté à savoir en translation par rapport à (X-X) et/ou en rotation autour de (X-X).

Il est bien entendu que l'instrument 15 est tout type d'instrument chirurgical connu en soi et apte à être inséré dans un trocart 9.

Un second capteur d'effort 17, de type connu en soi et couramment disponible dans le commerce, par exemple un capteur connu sous le nom de ATI Nano43 (marque déposée), est disposé entre l'extrémité 16 d'un bras robotisé 7 et l'instrument 15.

Le choix de la forme et des fonctions du second capteur d'efforts 17 est indépendant du choix de la forme et des fonctions du premier capteur d'efforts 11.

Avantageusement, le second capteur 17 est de forme cylindrique, par exemple sous la forme d'un galet comportant un orifice central 18.

Pour connaître l'effort d'interaction entre l'instrument 15 et les organes internes du patient 6, il a été développé un estimateur basé sur les équations dynamiques faisant intervenir au niveau de la liaison entre le trocart 9 et l'instrument 15 des forces et moments de torsion.

De manière plus précise, en notant $W_{i \to j}$ le torseur, c'est-à-dire la force et le moment en un point arbitraire, des actions mécaniques exercées par le corps i sur le corps j et $W_{gravité \to i}$ le torseur représentant l'action du champs gravitationnel sur le corps i, il est possible d'effectuer une modélisation statique du trocart, en supposant que le système est en équilibre.

En effet, en négligeant les effets dynamiques, on détermine l'équation d'équilibre de l'instrument 15, à savoir :

$$\sum W_{extérieur \to instrument}=0=W_{second\_capteur\_d'efforts \to instrument}+W_{guide \to instrument}+W_{organe \to instrument}+W_{gravité \to instrument}$$

Toutefois, afin de prendre en compte les effets dynamiques, il est possible de disposer des capteurs permettant de mesurer ou d'estimer les accélérations des corps et d'utiliser des mesures conjointement avec un modèle des objets, pour compenser les effets inertiels, cette technique étant bien connue par l'homme de l'art.

On détermine ensuite l'équation d'équilibre du guide 12, à savoir :

$$\sum W_{extérieur \to guide}=0=W_{instrument \to guide}+W_{premier\_capteur\_d'efforts \to guide}+W_{gravité \to guide}$$

Le premier capteur d'efforts 10 permet de mesurer $W_{premier\_capteur \to guide}$ et le second capteur d'efforts 17 permet de mesurer $W_{second\_capteur \to instrument}$.

A partir des deux équations précédentes, il est possible de déterminer la force d'interaction de l'instrument 15 et des organes internes du patient 6.

En effet, on a :

$$W_{instrument \to organe}=W_{premier\_capteur \to guide}+W_{second\_capteur \to instrument}+W_{gravité}$$

Avec $W_{gravité}=W_{gravité \to guide}+W_{gravité \to instrument}$

Une fois que $W_{premier\_capteur \to guide}$ et $W_{second\_capteur \to instrument}$ ont été mesurés, on exprime $W_{premier\_capteur \to guide}$ dans la même base et au même point que la mesure $W_{second\_capteur \to instrument}$, la mise en oeuvre de cette estimation étant évidente pour l'homme de l'art.

Il sera alors par la suite calculé le torseur des efforts de gravité , à savoir

$$\hat{W}_{gravité}=\hat{W}_{gravité \to instrument}+\hat{W}_{gravité \to guide}$$

Ce calcul, basé sur un modèle poids, est évident pour l'homme de l'art.

Finalement, en exprimant tous les torseurs dans la base de mesure $W_{second\_capteur \to instrument}$ au point de mesure de $W_{second\_capteur \to instrument}$, il est ensuite estimé l'interaction de l'instrument 15 sur les organes internes du patient 6, c'est-à-dire :

$$\hat{W}_{instrument \to organe}=W_{second\_capteur\_d'efforts \to instrument}+W_{premier\_capteur\_d'efforts \to guide}+\hat{W}_{gravité}$$

Cette estimation est réalisée par un calculateur, de type connu en soi, et permet un affichage de la force exercée par l'instrument sur les organes internes au niveau de l'interface 2 à l'aide de moyens électriques de type connus en soi.

De plus, les paramètres physiques, tels que les masses et le centre de gravité, et les paramètres géométriques, tels que la position et l'orientation relative des capteurs d'efforts, la position de l'instrument 15 relativement au trocart 9, sont, soit connus a priori si un modèle a été identifié, soit issus d'une procédure de calibrage initiale, dont la mise en oeuvre est classique pour l'homme de l'art.

La figure 3 représente une vue éclatée d'un trocart associé à un capteur d'efforts et un translateur.

La figure 3 est une représentation alternative du dispositif de trocart selon l'invention où il est uniquement nécessaire d'incorporer un seul capteur d'efforts pour déterminer les forces d'interaction entre un instrument chirurgical et les organes internes d'un patient.

Dans la suite de la description, les mêmes éléments de référence par rapport à la figure 2 porteront les mêmes numéros de référence.

En effet, afin d'apprécier les efforts exercés par un instrument 15 sur les organes internes d'un patient 6, il est disposé sur un trocart 9 de type connu en soi un guide 12 sous la forme d'un élément tubulaire 13 et d'une plaque circulaire 14.

De manière avantageuse, le guide 12 se présente sous la forme d'un élément tubulaire 13 avec sur une de ses extrémités une plaque circulaire 14 perpendiculaire à l'axe longitudinale (X-X) de l'élément tubulaire 13.

Entre le guide 12 et le trocart 9 est disposé un capteur d'effort 19, du même type que ceux précédemment utilisés pour le trocart de la figure 2, c'est-à-dire sous la forme d'un galet présentant un orifice central 20 pour le passage de l'instrument 15 et du guide passif 12.

Ainsi, le capteur d'effort 19 est de type connu en soi et couramment disponible dans le commerce, par exemple un capteur connu sous le nom ATI Nano43 (marque déposée).

L'élément tubulaire 13 du guide 12 est inséré dans l'orifice central 20 du capteur d'efforts 19 et dans le trocart 9.

Un translateur 21 est disposé sur le plaque circulaire 14 du guide 12 et est apte à permettre le déplacement longitudinal selon (X-X) d'un instrument 15 (non représenté sur la figure 3 par mesure de clarté mais du même type que celui de la figure 2).

De manière avantageuse, le translateur 21 est de type connu en soi, par exemple un translateur à galets.

Le trocart 9 est directement mis en mouvement par l'extrémité 16 d'un bras robotisé 7.

De manière alternative, le trocart 9 peut être mis en mouvement par un système robotisé autonome pouvant incliner le trocart 9 selon des orientations différentes.

Ainsi, tout effort entre l'instrument 15 et les organes internes du patient 6 est retransmis par le mécanisme translateur 21 au capteur d'efforts 19.

De manière avantageuse, une commande à retour d'efforts, de type connue en soi par l'homme de l'art, a été développée pour permettre à partir d'un capteur externe 19 de contrôler les forces intra-corporelles malgré les frottements induits par le trocart 9.

Plus précisément, comme pour le trocart de la figure 2, il est noté, pour l'estimation de la force d'interaction entre l'instrument 15 et les organes internes 6, $W_{i \to j}$ le torseur, c'est-à-dire la force et le moment en un point arbitraire, des actions mécaniques exercées par le corps i sur le corps j et $W_{gravité \to i}$ le torseur représentant l'action du champs gravitationnel sur le corps i, il est possible d'effectuer une modélisation statique du trocart, en supposant que le système est en équilibre.

En effet, aux vitesses utiles en chirurgie, les effets inertiels des accélérations peuvent être négligés.

Afin de modéliser et d'estimer les différentes forces du trocart 9, il est déterminé les équations d'équilibre de l'instrument 15, du translateur 21 et du guide 12, soit :

- Equation d'équilibre de l'instrument 15 :

$$\sum W_{extérieur \to instrument} = 0 = W_{translateur \to instrument} + W_{guide \to instrument} + W_{organe \to instrument} + W_{gravité \to instrument}$$

- Equation d'équilibre du translateur 21 :

$$\sum W_{extérieur \to translateur} = 0 = W_{instrument \to translateur} + W_{guide \to translateur} + W_{gravité \to translateur}$$

- Equation d'équilibre du guide 12 :

$$\sum W_{extérieur \to guide} = 0 = W_{translateur \to guide} + W_{instrument \to guide} + W_{capteur\_d'efforts \to guide} + W_{gravité \to guide}$$

Il est à noter que $W_{capteur\_d'efforts \to guide}$ est la force mesurée par le capteur d'efforts 19.

On cherche à estimer la force d'interaction de l'instrument 15 avec les organes internes du patient 6, c'est-à-dire $W_{organe \to instrument}$.

En combinant les trois équations précédentes, on obtient :

$$W_{capteur\_d'efforts \to guide} = - W_{translateur \to guide} - W_{instrument \to guide} - W_{gravité \to guide}$$

$$W_{capteur\_d'efforts \rightarrow guide} = W_{guide \rightarrow translateur} + W_{guide \rightarrow instrument} - W_{gravité \rightarrow guide}$$

or on a :

$$W_{guide \rightarrow translateur} = -W_{instrument \rightarrow translateur} - W_{gravité \rightarrow translateur}$$

et

$$W_{guide \rightarrow instrument} = -W_{translateur \rightarrow instrument} - W_{organe \rightarrow instrument} - W_{gravité \rightarrow instrument}$$

Ainsi, on obtient finalement :

$$W_{capteur\_d'efforts \rightarrow guide} = W_{instrument \rightarrow organe} - \left( W_{gravité \rightarrow translateur} + W_{gravité \rightarrow instrument} + W_{gravité \rightarrow guide} \right)$$

Ainsi, les efforts mesurés par le capteur 19 correspondent aux efforts internes entre l'instrument 15 et les organes internes du patient 6, au poids de l'ensemble instrument 15/ guide passif 12 / translateur 21 près.

Par ailleurs, il est à noter que les frottements entre le guide passif 12 et l'instrument 15 ainsi que les interactions entre la paroi abdominale et le trocart 9 n'interviennent pas dans la mesure.

Ainsi, pour estimer les interactions entre l'instrument 15 et les organes internes du patient 6, il convient tout d'abord de mesurer le torseur délivré par le capteur d'effort 19, à savoir $W_{capteur\_d'efforts \rightarrow guide}$.

Il est ensuite nécessaire de calculer le torseur des efforts de gravité, à savoir :

$$\hat{W}_{gravité} = W_{gravité \rightarrow translateur} + W_{gravité \rightarrow instrument} + W_{gravité \rightarrow guide}$$

Il est alors possible d'estimer l'interaction entre l'instrument 15 et les organes internes du patient 6 de par l'équation :

$$\hat{W}_{instrument \rightarrow organe} = W_{capteur\_d'efforts \rightarrow guide} + \hat{W}_{gravité}$$

Pour calculer le torseur des efforts de gravité, plusieurs méthodes sont couramment utilisées :

-    Soit le modèle poids (masse et lieu du centre de gravité) de l'instrument 15, du translateur 21 et du guide 12 est parfaitement connu.
    Dans ce cas, on calcule le torseur de gravité à partir de la mesure de l'orientation du trocart 9, réalisée à partir de capteurs de position disposés sur le bras robotisé 16 directement relié au trocart 9, et à partir de la mesure de la position de l'instrument 15 par rapport au guide 12, réalisée à partir de capteurs de position disposés sur le translateur 21, cette méthode de mesure étant évidente pour l'homme de l'art.
-    Soit il n'est pas connu un ou plusieurs paramètres requis pour le calcul à la base du modèle.
    Dans ce cas, un calibrage préalable à l'opération est réalisé. Pour cela, on place le système dans différentes configurations géométriques à l'aide du translateur 21 et de l'extrémité 16 du bras robotisé 7, tout en veillant que l'instrument 15 ne soit pas en contact avec les organes internes du patient 6.
    Il est alors possible de construire, soit une table de correspondance, soit d'identifier les paramètres du modèle poids, selon un mode opératoire bien connu par l'homme de l'art.

Il est également possible d'exprimer le torseur des efforts exercés par l'instrument 15 sur les organes internes du patient 6 dans une base liée à l'instrument 15, et non au capteur d'efforts 19, et dans un point correspondant à l'extrémité de l'instrument 15, et non en un point lié au capteur d'efforts 19.

Dans ce cas, il suffit de connaître la position relative de l'instrument par rapport au capteur 19, ce qui se calcule selon des méthodes classiques pour l'homme de l'art.

Ainsi, il est possible à partir de capteurs d'efforts (10, 17, 19) disposés à l'extérieur d'un trocart 9 de déterminer les forces d'interaction entre un instrument chirurgical 15 et un organe interne d'un patient 6.

L'estimation de la force d'interaction entre l'instrument chirurgical 15 et les organes internes d'un patient 6 est réalisée à partir des torseurs mesurés par les capteurs d'efforts (10, 17, 19), un calculateur, de type connu en soi, permettant un affichage instantané de la force exercée par l'instrument 15 sur les organes internes du patient 6 au niveau de l'interface 2.

De manière avantageuse, le chirurgien peut, à partir de l'interface 2, déterminer la force maximale qu'il veut appliquer sur les organes internes du patient 6 et qu'il ne pourra dépasser.

Cette limitation de l'effort appliqué aux organes internes 6 permet de garantir qu'un geste brutal incontrôlé d'une force élevée n'aura pas d'incidence sur les organes internes du patient 6.

De manière avantageuse, l'interface 2 présente des moyens de contrôle de la force appliquée par l'instrument et/ou des moyens de restitution de la force exercée par l'instrument au chirurgien par l'intermédiaire des bras de commande 4.

**Revendications**

1. Dispositif de trocart (9) pour le passage d'un instrument chirurgical (15), **caractérisé en ce qu'**il comporte des moyens de mesure (10, 17, 19) de l'effort exercé par ledit instrument (15) sur les organes internes d'un patient (6), lesdits moyens de mesure se présentant sous la forme d'au moins un capteur d'efforts (10, 19) disposé entre ledit trocart (9) et un guide (12).

2. Dispositif selon la revendication 1, **caractérisé en ce que** ledit capteur d'efforts (10.19), est disposé sur ledit trocart (9).

3. Dispositif selon la revendication 1 ou 2, **caractérisé en ce que** ledit capteur (10, 19) se présente sous la forme d'un galet avec un orifice central (11, 20).

4. Dispositif selon l'une des revendications précédentes, **caractérisé en ce que** ledit guide (12) se présente sous la forme d'un élément tubulaire (13) d'axe longitudinal (X-X) comportant une plaque circulaire (14), perpendiculaire à (X-X), à une de ses extrémités.

5. Dispositif selon la revendication 3 ou 4, **caractérisé en ce que** ledit guide (12) est inséré dans ledit orifice central (11, 20) dudit capteur d'efforts (10, 19) et ledit dispositif de trocart (9).

6. Dispositif selon l'une des revendications précédentes, **caractérisé en ce que** ledit instrument (15) est mis en mouvement par un bras robotisé (7).

7. Dispositif selon la revendication 6, **caractérisé en ce qu'**un second capteur d'efforts (17) est disposé entre l'extrémité (16) dudit bras robotisé (7) et ledit instrument chirurgical (15).

8. Dispositif selon l'une des revendications 1 à 7, **caractérisé en ce que** ledit instrument (15) est mis en mouvement par un translateur (21).

9. Dispositif selon la revendication précédente, **caractérisé en ce que** ledit translateur (21) est disposé sur ledit guide (12).

10. Dispositif selon la revendication 8 ou 9, **caractérisé en ce que** ledit translateur (21) est un translateur à galet.

11. Dispositif selon l'une des revendications 9 ou 10, **caractérisé en ce qu'**il est mis en mouvement par l'extrémité (16) d'un bras robotisé (7).

12. Dispositif selon la revendication 6, 7 ou 11, **caractérisé en ce que** le déplacement dudit bras robotisé (7) est commandé à partir d'une interface (2).

**Claims**

1. A trocar device (9) for the passage of a surgical instrument (15), **characterised in that** it comprises means (10, 17, 19) for measuring the load exerted by said instrument (15) on the internal organs of a patient (6), said measuring means taking the form of at least one load sensor (10, 19) disposed between said trocar (9) and a guide (12).

2. A device according to claim 1, **characterised in that** said load sensor (10, 19) is disposed on said trocar (9).

3. A device according to claim 1 or claim 2, **characterised in that** said sensor (10, 19) takes the form of a roller with a central orifice (11, 20).

4. A device according to one of the preceding claims, **characterised in that** said guide (12) takes the form of a tubular member (13) with a longitudinal axis (X-X) comprising a circular plate (14), perpendicular to (X-X), at one of its ends.

5. A device according to claim 3 or claim 4, **characterised in that** said guide (12) is inserted into said central orifice (11, 20) of said load sensor (10, 19) and said trocar device (9).

6. A device according to one of the preceding claims, **characterised in that** said instrument (15) is set in motion by a robotic arm (7).

7. A device according to claim 6, **characterised in that** a second load sensor (17) is disposed between the end (16) of said robotic arm (7) and said surgical instrument (15).

8. A device according to one of claims 1 to 7, **characterised in that** said instrument (15) is set in motion by a translatory device (21).

9. A device according to the preceding claim, **characterised in that** said translatory device (21) is disposed on said guide (12).

10. A device according to claim 8 or claim 9, **characterised in that** said translatory device (21) is a roller translatory device.

11. A device according to one of claims 9 or 10, **characterised in that** it is set in motion by the end (16) of a robotic arm (7).

12. A device according to claim 6, claim 7 or claim 11, **characterised in that** displacement of said robotic arm (7) is controlled from an interface (2).


**Patentansprüche**

1. Trokar (9) für die Durchführung eines chirurgischen Instrumentes (15), **dadurch gekennzeichnet, dass** es Messmittel (10, 17, 19) für die mit dem Instrument (15) auf die Innenorgane des Patienten (6) ausgeübte Kraft aufweist, wobei die Messmittel in Form wenigstens eines Kraft-Sensors (10, 19) vorliegen, der zwischen dem Trokar (9) und einer Führung (12) angeordnet ist.

2. Vorrichtung gemäß Anspruch 1, **dadurch gekennzeichnet, dass** der Kraft-Sensor (10, 19) auf dem Trokar (9) angeordnet ist.

3. Vorrichtung gemäß Anspruch 1 oder 2, **dadurch gekennzeichnet, dass** der Sensor (10, 19) in Form einer Rolle mit einer Mittelöffnung (11, 20) vorliegt.

4. Vorrichtung gemäß einem der vorausgegangenen Ansprüche, **dadurch gekennzeichnet, dass** die Führung (12) in Form eines rohrförmigen Elements (13) mit Längsachse (X-X) vorliegt, welches an einem seiner Enden senkrecht zu (X-X) eine kreisförmige Scheibe (14) aufweist.

5. Vorrichtung gemäß Anspruch 3 oder 4, **dadurch gekennzeichnet, dass** die Führung (12) in die Mittelöffnung (11, 20) des Kraft-Sensors (10, 19) und des Trokars (9) eingesetzt wird.

**6.** Vorrichtung gemäß einem der vorausgegangenen Ansprüche, **dadurch gekennzeichnet, dass** das Instrument (15) durch einen Roboterarm (7) bewegt wird.

**7.** Vorrichtung gemäß Anspruch 6, **dadurch gekennzeichnet, dass** ein zweiter Kraft-Sensor (17) zwischen dem Ende (16) des Roboterarms (7) und dem chirurgischen Instrument (15) angeordnet wird.

**8.** Vorrichtung gemäß einem der Ansprüche 1 bis 7, **dadurch gekennzeichnet, dass** das Instrument (15) durch einen Übertrager (21) bewegt wird.

**9.** Vorrichtung gemäß dem vorausgegangenen Anspruch, **dadurch gekennzeichnet, dass** der Übertrager (21) auf der Führung (12) angeordnet ist.

**10.** Vorrichtung gemäß Anspruch 8 oder 9, **dadurch gekennzeichnet, dass** der Übertrager (21) ein Rollen-Übertrager ist.

**11.** Vorrichtung gemäß einem der Ansprüche 9 oder 10, **dadurch gekennzeichnet, dass** diese durch das Ende (16) eines Roboterarms (7) bewegt wird.

**12.** Vorrichtung gemäß Anspruch 6, 7 oder 11, **dadurch gekennzeichnet, dass** das Versetzen des Roboterarms (7) von einer Schnittstelle (2) gesteuert wird.

**Fig. 1**

**Fig. 2**

**Fig. 3**